# EUROPÄISCHE PATENTSCHRIFT

(11) **EP 0 596 483 B1**
(45) Veröffentlichungstag und Bekanntmachung des Hinweises auf die Patenterteilung: **29.05.1996**
(21) Anmeldenummer: 93117856.0
(22) Anmeldetag: 04.11.1993
(51) Int. Cl.: C07C 69/06, C07C 67/36

(54) **Verfahren zur Herstellung von Methylformiat**
Process for the preparation of methyl formiate
Procédé de fabrication du formiate de méthyle

(30) Priorität: 05.11.1992 DE 4237379
(43) Veröffentlichungstag der Anmeldung: 11.05.1994
(73) Patentinhaber: Salzgitter Anlagenbau Zweigniederlassung der Preussag Anlagenbau GmbH, D-38223 Salzgitter (DE); BORISLAWER WISSENSCHAFTS- UND FORSCHUNGSINSTITUT BNII " SYNTHEZ", Borislaw 293 760 (UA)
(72) Erfinder: Sobotta, Georg, Dr.-Ing., W-38239 Salzgitter (DE); Pazderski, Juri Antonowitsch Prof. Dr., UA-293760 Borislaw, Gebiet Lwow (UA); Skatschko, Wladimir Petrowitsch Dr., UA-293760 Borislaw, Gebiet Lwow (UA); Moiseev, Illa Iosifovitsch Prof. Dr., UA-107392, Rossija, Moskau, B-392 (UA); Tagajew, Oleg Alexejewitsch, Dr., UA- 293760 Borislaw, Gebiet Lwow (UA)
(74) Vertreter: Drömer, Hans-Carsten

(56) Entgegenhaltungen:
- EP-A- 0 104 875
- FR-A- 2 528 830

## Beschreibung

Die Erfindung ist dem Gebiet der Technologie der industriellen organischen Synthese, speziell den Methoden zur Erzeugung von Methylformiat, zuzuordnen.
Der Wert des Endproduktes besteht in der Vielseitigkeit seiner chemischen Eigenschaften.
Methylformiat kann man als Kohlenmonoxid enthaltenden Donator zur Herstellung von Carbonsäuren und deren Ester benutzen, als Formylierungsagens, und außerdem ist es ein Zwischenprodukt im Prozeß der Herstellung des Formamids und der Ameisensäure, die ein wichtiges Konservierungsmittel für landwirtschaftliche Futtermittel ist.
Die Methode zur Erzeugung vom Methylformiat basiert auf der Carbonylierung vom Methanol mit Kohlenmonoxid unter Druck und im Beisein von Alkaliformiat (DE-PS 495935, Veröffentl. 27.03.1930). Die industrielle Anwendung der Methode wird durch nachfolgende Umstände erschwert:
- Die Notwendigkeit zur Herstellung einer ausreichend großen Oberfläche und Vergrößerung der Verweilzeit zwischen gasförmigem Kohlenmonoxid und der flüssigen methanolischen Lösung des Katalysators als Voraussetzung für eine hohe Ausbeute,
- die Entfernung des Katalysators aus dem Reaktionsapparat und das Zusetzen der Armaturen, der Rohrleitungen und des Reaktors mit den aufgezeigten Produkten,
- die unproduktiven Verluste des Katalysators dadurch, daß sich dieser mit den Zerfallsprodukten vermengt.

In bekannten Methoden der Intensivierung des Reaktionsprozesses wendet man verschiedene Varianten für die Herstellung des Kontaktes zwischen dem Gas (CO) und der Flüssigkeit (Methanol) an (DE-PS 863046, Veröffentl. 13.01.1953; DE-OS 2243811, Veröffentl. 04.04.1974; DE-OS 2710726, Kl. CO7C 69/06, Veröffentl. 15.09.1977; Urheberschein UdSSR Nr. 1223598, Kl. CO7C 69/06, angekündigt 08.02.1982).

Die mit den besseren Methoden erzielten Ergebnisse zeigen, daß der Prozeß der Carbonylierung im kinetischen Bereich abläuft, d. h., eine Intensivierung des Prozesses ist durch eine weitere Erhöhung der Kontaktoberfläche nicht erreichbar.
Eine Erhöhung der Geschwindigkeit der Methylformiatsynthese auf der Basis Methanol und Kohlenmonoxid kann nur erzielt werden durch Erhöhung der Geschwindigkeit der chemischen Reaktion infolge einer Veränderung der Eigenschaft des Reaktionsgemisches.
Von der technischen Seite und den erzielten Resultaten kommt der vorliegenden Erfindung am nächsten die Erzeugung von Methylformiat durch Carbonylierung von Methanol im Beisein alkalischer Alkoholate und Zusätze von Oligomeren mit der Formel HO(CH2CHRO)ₚH, wobei R = H oder CH3 und p = 2 bis 1000 ist sowie Zusätze von Pyridin im Reaktionsgemisch der Carbonylierung (DE-PS 3221239, Kl. CO7C 69/06, Veröffentl. 08.12.1983).
Der Prozeß läuft bei einer Temperatur von 60 - 120 Grad Celsius und einem Druck des CO = 3,0 MPa ab, die Konzentration des Natriummethylats beträgt 2 bis 6 Ma. -% (Ma.-% entspricht Gew.%). Die Zugabe von Oligomeren hydratisierter Olefine, wie zum Beispiel Polyethylenglykolen, verlangsamt das Absetzen der Zerfallsprodukte des Katalysators innerhalb der Reaktionsapparatur, wodurch sich die Gefahr der Verstopfung verringert und sich so die Möglichkeit ergibt, den festen Niederschlag an einem definierten Punkt der Anlage abzutrennen (Filter).

Bei der gleichzeitigen Anwendung von Pyridin mit Oligomeren hydratisierter Olefine und Natriummethylat im katalytischen System der Carbonylierung von Methanol ist eine gewisse Erhöhung der Produktivität des Prozesses festzustellen (Ausbeute des Methylformiates 110 bis 200 Gramm/Liter je Stunde).
Der wesentliche Nachteil der bekannten technischen Lösungen liegt im hohen spezifischen Verbrauch des angewandten Katalysators Natriummethylat (4,14 bis 5,75 Kilogramm je Tonne Methylformiat) infolge seines Absetzens mitsamt seinen Zersetzungsprodukten in den Filtern der Carbonylierungsanlagen.

Eine gewisse Erhöhung der Produktivität der gezeigten Methode rechtfertigt nicht die Anwendung des hochgiftigen Pyridins als Bestandteil des katalytischen Systems. Die Unzulänglichkeit der "Prototyp"-Methode liegt in der geringen Prozeßgeschwindigkeit.

Eine wesentliche Unzulänglichkeit bekannter technischer Lösungen besteht darin, daß sich im Verlaufe der Prozeßreaktionen ascheähnliche Produkte bilden, die den Abtrennungsprozeß durch Filtration der Produkte, die den Katalysator desaktivieren, erschweren.
Solche ascheähnlichen Produkte einer ungeregelten Zusammensetzung entstehen im Ergebnis der Kondensationsreaktionen der Oligomere hydratisierter Olefine, wie Polyethylenglykole, und der Carbonylierungsprozesse.

Das Ziel der erfindungsgemäßen Methode zur Erzeugung von Methylformiat besteht in der Verringerung des Katalysatorverlustes und der Intensivierung des Reaktionsprozesses. Das gesteckte Ziel erreicht man dadurch, daß die mit Natrium- oder Kaliummethylat katalysierte Reaktion von Methanol mit CO unter Druck und bei erhöhter Temperatur in Anwesenheit von 0,005 bis 0,15 Mol/Liter Natrium- oder Kaliumperfluoralkansulfonat der allgemeinen Formel

CF₃CF₂(OCFXCF₂)ₚOCF₂CF₂SO₃M

mit
p = 0 bis 2
X = F, CF3
M = Na, K
sowie 1 bis 10 Ma. -% starker organischer Basen mit pKa > 8,7 durchgeführt wird, wobei die Konzentration des Methylformiates in den Produkten der Carbonylierung des Methanols im Bereich von 15 bis 28 Ma. -% liegt.
Die Nutzung der aufgezeigten Verbindungen als Bestandteile des katalytischen Systems ermöglicht, die Effektivität des Produktionsprozesses zu erhöhen (Ausbeute des Methylformiates beträgt bezogen auf 1 Liter des Reaktionsgemisches 270,2 Gramm/Stunde), wobei sich die Löslichkeit von Natrium- oder Kaliummethylat und seiner Zersetzungsprodukte (Natrium- oder Kaliumformiat, Natrium- oder Kaliumcarbonat, Natrium- oder Kaliumhydrogencarbonat) verändert; im Ergebnis dessen verändern sich die Mengenverhältnisse dieser Salze in der flüssigen Lösung und in der festen Ablagerung in den Filtern der Carbonylierungsanlage, und es verringert sich der spezifische Verbrauch des Katalysators (1,52 Kilogramm je Tonne Methylformiat).
Weiterhin ist keine Bildung von ascheähnlichen Produkten zu verzeichnen, was sich positiv von den vorgeschlagenen Methoden bekannter technischer Lösungen unterscheidet. Wesentlich ist ebenso, daß die Konzentration des Methylformiates in den Carbonylierungsprodukten im Bereich von 15 bis 28 Ma. -% aufrechterhalten wird.

Diese technische Lösung verstärkt den Effekt der Erhöhung der Löslichkeit der Methylate, der Formiate, der Carbonate und der Hydrogencarbonate der Alkalimetalle im Reaktionsgemisch, was durch die oben vorgeschlagenen Zusätze zum katalytischen System der Carbonylierung von Methanol bewirkt wird, und somit wird der Katalysatorverbrauch verringert.

Es ist optimal, 0,01 bis 0,1 Mol/Liter des Kaliumsalzes der
5-Methyl-3,6-dioxa-perfluoroctansulfonsäure als eine der vorgeschlagenen Komponenten eines Natrium- oder Kalium-Oxa-perfluoralkansulfonates für das katalytische System der Methylformiat-Synthese einzusetzen,

CF₃CF₂OCF(CF₃)CF₂OCF₂CF₂SO₃K

wodurch sich der spezifische Verbrauch des Katalysators auf 1,52 Kilogramm/Tonne Methylformiat verringert.

Diesbezüglich hat die Art des Metalles (Na,K) oder der Substituenten (F,CF3) in den aufgezeigten Verbindungen keinen bedeutenden Einfluß auf den Prozeß der Carbonylierung von Methanol.
Die obere Grenze der Zahl der oligomeren Gruppen - OCFXCF₂ - im Molekül ist p=2. Bei p=3 tritt eine Durchsatzreduzierung aufgrund der Verringerung des Hauptreaktanten (Methanol) in der Ausgangsreaktionslösung ein.
Die in der vorgeschlagenen Methode angewandten Natrium- oder Kalium-Oxaperfluoralkansulfonate der allgemeinen Formel
C₂F₅(OCFXCF₂)ₚOC₂F₄SO₃M erhält man mit der Methode, die im Patent USA Nr. 3555080 (Kl. CO7C 143/00, Veröffentl. 25.08.69) beschrieben ist.
Als starke organische Basen mit pKa > 8,7 können verschiedene Amine eingesetzt werden:
Morpholin
N-Methyl-α-Pyrrolidon
Guanidin und seine Derivate
wobei die Konzentration der genannten Komponenten (nicht mehr als 10 Ma. -%) von der Produktivität des Prozesses bestimmt wird.
Optimal ist eine Anwendung von 3 bis 8 % N-Methyl-α-Pyrrolidon (pKa=11,2) oder 2 bis 8 % Guanidin oder seiner Derivate (pKa=13,6), durch deren Vorhandensein sich eine erhöhte Produktivität des Reaktionsvolumens bezogen auf Methylformiat ergibt (entsprechend 287,1 bis 296,7 Gramm/Stunde oder 302,5 bis 318,3 Gramm/Stunde).
Das Neue an der vorliegenden Erfindung besteht in der Anwendung von Natrium- oder Kalium-Oxaperfluoralkansulfonaten als Komponenten des katalytischen Systems der Carbonylierung von Methanol sowie die Anwendung starker organischer Basen mit pKa > 8,7. Neu ist ebenso die Forderung, die Konzentration des Methylformiates in den Produkten in den Grenzen von 15,0 bis 28,0 Ma.-% zu halten.
Die Methode zur Erzeugung von Methylformiat entsprechend der vorgeschlagenen Erfindung kann man in einem der beliebigen Apparatetypen durchführen, die in den Patenten dargelegt sind: DE-PS 863046, DE-OS 2243811, DE-OS 2718726 (Kl. CO7C 69/06) oder Urheberschein UdSSR Nr. 1223598 (Kl. CO7C 68/06).
Beispiele für die Durchführung der Methode in den Apparaten sind in DE-OS 32221239 (Kl. CO7C 69/06, Veröffentl. 08.12.83) beschrieben.

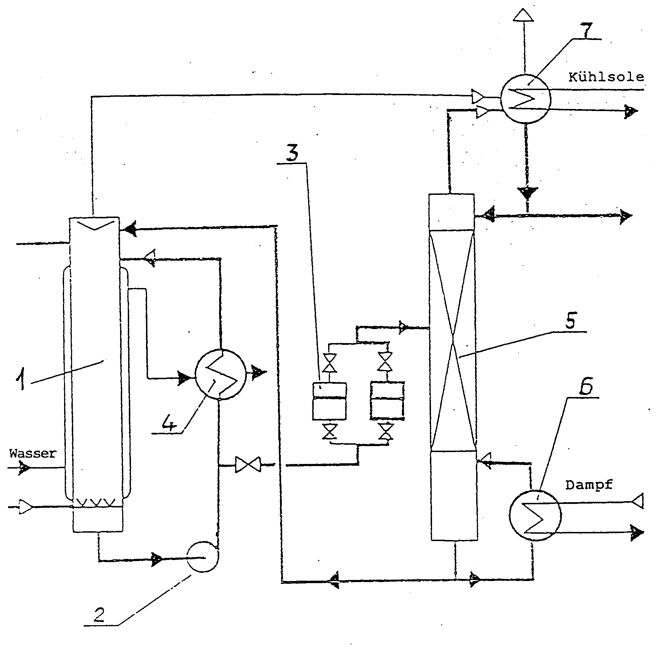

In der beigefügten Figur ist eine Anlage für die Methylformiaterzeugung dargestellt, die aus einem beheizten Reaktor 1 mit Zirkulationspumpe 2 für die effektive Vermischung von CO mit Methanol, einem Filter 3, einem äußeren Wärmetauscher 4 und einer Rektifikationskolonne 5 mit Verdampfer 6 und dem Dephlegmator-Kondensator 7 besteht. Der Carbonylierungsreaktor 1 wird vor der Inbetriebnahme beheizt, danach führt man in den oberen Teil methanolische Natrium-Methylat-Lösung mit den zugesetzten Komponenten des katalytischen Systems ein, und in den unteren Teil des Reaktors wird unter Druck CO eingeleitet. Das Gas-Flüssigkeits-Gemisch aus dem unteren Teil wird mittels Pumpe 2 über den äußeren Wärmetauscher 4 in den oberen Teil des Reaktors geleitet. Ein Teil des Produktstromes aus dem Reaktor, welcher der Menge des sich bildenden Methylformiates entspricht, wird mit der Pumpe 2 über den Filter 3 zur Trennung in die Rektifikationskolonne 5 mit Verdampfer 6 und Dephlegmator-Kondensator 7 gegeben.

Als Destillat der Kolonne erhält man Methylformiat, im Sumpf verbleibt eine Lösung des katalytischen Systems der Carbonylierung mit dem nicht umgesetzten Methanol, die in den Reaktor 1 zur Methylformiatsynthese zurückgeführt wird.
Die Ablagerung von Zerfallsprodukten des Katalysators Natriummethylat werden periodisch vom Filter 3 entfernt und die Zusammensetzung bestimmt. Danach wird der Filter 3 mit Methanol gespült und mit trockenem Stickstoff durchgeblasen.
Die Abgase aus dem Reaktor 1, die CO-, NO- und Sauerstoffanteile sowie andere Gase enthalten, werden über den Kondensator 7 einer Verbrennung zugeführt.
Das Wesen der Erfindung wird an folgenden Beispielen demonstriert:

### Beispiel 1

Der Prozeß zur Erzeugung von Methylformiat entsprechend der vorgeschlagenen Methode wird in einer Apparatur durchgeführt, die in DE-PS 3221239 beschrieben ist.

Die Reaktionen von Methanol mit Kohlenmonoxid werden in einem zylindrischen Reaktor mit Fassungsvermögen von 10 Litern, ausgerüstet mit einer thermostatischen Ummantelung und einer Zirkulationspumpe für eine effektivere Vermischung des Gas-Flüssigkeits-Gemisches durchgeführt. Die flüssigen Produkte der Carbonylierung passieren den Filter und werden in die Rektifikationskolonne geleitet, in der man Methylformiat als Destillat erhält. Das Sumpfprodukt (Methanollösung des Katalysators) wird mit frischem Methanol versetzt und in den Reaktor zur Carbonylierung zurückgeführt. In den auf 80 Grad Celsius erhitzten Reaktor werden 13587,2 Gramm/Stunde des Ausgangsgemisches für die Carbonylierung gegeben.

Dieses enthält:
2,50 Ma. -% Natriummethylat (1,06 Ma.-% bezogen auf Na)
0,90 Ma. -% Natriumsalze (Reaktionsprodukte Methylats)
8,00 Ma. -% N-methyl-α-Pyrrolidon
1,25 Ma. -% (0,021 Mol/Liter) Natriumsalz der
Perfluor-5-methyl-3,6-dioxa-perfluoroctansulfonsäure

C₂F₅OCF(CF₃)CF₂OC₂F₄SO₃Na

87,35 Ma.-% Methanol(Wasseranteil geringer als 0,004 Ma.-%)
Zum Erreichen eines Partialdruckes von 3,0 MPa führt man in den Reaktor 1300 Gramm/Stunde des gasförmigen Kohlenmonoxids (Konzentration des CO 99,2 %), welches 0,13 Ma.-% CO₂ und nicht mehr als 0,005 Ma.-% Wasser enthält (Restbeimengungen von Stickstoff, Methan, Sauerstoff). Für eine intensivere Vermischung des Gases mit der Flüssigkeit wird das Gemisch mit der Zirkulationspumpe aus dem unteren in den oberen Teil des Reaktors mit einer Geschwindigkeit von 60 Liter/Stunde umgepumpt.
Aus dem Reaktor werden 14851,6 Gramm/Stunde flüssige Produkte der Carbonylierung entnommen, die nach der chromatographischen und chemischen Analyse folgende Stoffe enthalten:
18,20 Ma.-% Methylformiat
70,22 Ma.-% Methanol
2,26 Ma.-% Natriummethylat
7,32 Ma.-% N-methyl-α-Pyrrolidon
1,14 Ma.-% Natriumsalz der Perfluor-5-methyl-3,6-dioxa-perfluoroctansulfonsäure
0,86 Ma.-% Natriumformiat, -carbonat und -hydrogencarbonat
Die Natriumsalze, Produkte der Reaktion des Natriummethylates, werden vom Filter entfernt, dabei erhält man 5,308 Gramm/Stunde Salze, die entsprechend der chemischen Analyse folgende Zusammensetzung haben:
37,60 Ma.-% Natriumformiat
42,12 Ma.-% Natriumhydrogencarbonat
14,49 Ma.-% Natriumcarbonat
5,65 Ma.-% Natriummethylat
Der Rest besteht aus Zusatzkomponenten des katalytischen Systems (N-Methyl-α-Pyrrolidon und Natriumsalz der Perfluor-5-methyl-3,6-dioxa-perfluoroctansäure).

Das Filtrat wird mit Hilfe der Rektifikation getrennt, und das Sumpfprodukt wird mit frischem Methanol in den Reaktor zur Carbonylierung zurückgeführt. Als Destillat erhält man
2702,5 Gramm/Stunden Methylformiat.
Somit erreicht man eine Produktivität von 270,25 Gramm/Stunde Methylformiat je Liter Reaktionsgemisch bei der Anwendung der in dieser Erfinung vorgeschlagenen Methode, wobei die Menge der sich bildenden Reaktionsprodukte des Natriummethylates 1,963 Kilogramm/Tonne Methylformiat beträgt, das heißt, der Verbrauch an Natriummethylat (bezogen auf 100 % CH₃ONa) ist 1,538 Kilogramm je Tonne Methylformiat.

### Beispiel 1 a (Wiedergabe des Vergleichsbeispiels, Prototyps)

Die Synthese von Methylformiat durch Carbonylierung von Methanol wird in einer Apparatur durchgeführt, die im Beispiel 1 beschrieben wurde und DE-PS 3221239 entspricht.
Die Bedingungen zur Durchführung der Methode des Prototyps entsprechen denen, die im Beispiel 3, DE-PS 3221239, aufgezeigt sind.
In den auf 80 Grad Celsius erwärmten Reaktor gibt man 3269 Gramm/Stunde des Ausgangsgemisches für die Carbonylierung von Methanol, welches enhält:
5,87 Ma.-% Natriummethylat (2,5 Ma.-% bezogen auf Na)
0,08 Ma.-% Natriumsalze (Reaktionsprodukte des Methylats)
5,00 Ma.-% Polyethylenglykol (R=H, p=1000)
5,000 Ma.-% Pyridin
0,02 Ma.-% Wasser
84,03 Ma.-% Methanol
Zum Erreichen eines Partialdruckes von 3,0 MPa führt man in den Reaktor 1000 Gramm/Stunde des gasförmigen 99,2 prozentigen CO ein, welches 0,13 Ma.-% CO₂ und weniger als 0,005 Ma.-% Wasser enthält (der Rest sind Beimengungen von Stickstoff, Methan und Sauerstoff).
Für eine intensivere Vermischung des Gases mit der Flüssigkeit wird das Gemisch mit der Zirkulationspumpe aus dem unteren in den oberen Teil des Reaktors mit einer Geschwindigkeit von 60 Litern/Stunde umgepumpt.
Aus dem Reaktor werden 4200 Gramm/Stunde der Carbonylierungsprodukte entnommen, die nach der chromatographischen und chemischen Analyse folgende Stoffe enthalten:
47,50 Ma.-% Methylformiat
40,13 Ma.-% Methanol
4,38 Ma.-% Natriummethylat
3,89 Ma.-% Polyethylenglykol
3,89 Ma.-% Pyridin
0,21 Ma.-% Natriumcarbonat, Natriumhydrogencarbonat und Natriumformiat
Die Natriumsalze, Produkte der Reaktion des Natriummethylates, setzen sich im Filter ab, wobei man 12,03 Gramm/Stunde Feststoff mit folgender Zusammensetzung entsprechend der chemischen Analyse erhält:
23,03 Ma.-% Natriumformiat
19,62 Ma.-% Natriumhydrogencarbonat (=Natriumbicarbonat)
6,75 Ma.-% Natriumcarbonat
38,15 Ma.-% Natriummethylat
12,45 Ma.-% Polyethylenglykol und aschähnlicher Produkte mit unregelmäßiger Zusammensetzung
Das Filtrat wird mit Hilfe der Rektifikation getrennt, das Sumpfprodukt wird mit frischem Methanol vermischt und zum Reaktor zur Carbonylierung zurückgeführt.
Als Destillat erhält man 1995 Gramm/Stunde Methylformiat. Somit erreicht man eine Produktivität von 199,5 Gramm/Stunde Methylformiat je Liter Reaktionsgemisch bei der Anwendung der Methode des "Prototyps", aber die Menge der sich bildenden Reaktionsprodukte des Natriummethylates beträgt 6,03 Kilogramm je Tonne Methylformiat, das heißt, der spezifische Verbrauch von Natriummethylat (bezogen auf 100 % CH₃ONa) ist gleich 4,58 je Tonne Methylformiat.

Aus dem Vergleich der Kennziffern der Produktivität der Prozesse, des Verbrauches des Katalysators und der Menge des sich bildenden Niederschlages bei der Durchführung der vorgeschlagenen Methode und der "Prototyp"-Methode (Vergleichsbeispiel 1 a) wird ersichtlich, daß selbst bei einer niedrigen Konzentration des Katalysators Natriummethylat (2,5 Ma.-% im Vergleich zu 5,87 Ma.-% beim "Prototyp") eine höhere Produktivität je Liter Reaktionsvolumen erhalten wird (die Ausbeute an Methylformiat ist 270,25 Gramm/Stunde im Vergleich zu 199,5 Gramm/Stunde beim "Prototyp"). Gleichzeitig verringert sich der Verbrauch an Natriummethylat (1,52 Kilogramm/Tonne im Vergleich zu 4,58 Kilogramm/Tonne Methylformiat) und die Menge der Ablagerungen - Reaktionsprodukte des Natriummethylates - (1,963 Kilogramm/Tonne im Vergleich zu 6,03 Kilogramm/Tonne beim "Prototyp").

### Beispiele 2 bis 14 (siehe Tabelle)

### Einfluß der Zusammensetzung des katalytischen Systems

Die Apparateanordnung und die technologischen Prozeßparameter entsprechen vollständig denen im Beispiel 1. Variiert wird nur die Zusammensetzung des katalytischen Systems der Carbonylierung von Methanol, wobei nicht nur verschiedene Mengen eingesetzt werden, sondern auch verschiedene chemische Verbindungen einer Klasse als Komponenten des katalytischen Systems.
Die Untersuchungsergebnisse sind in der Tabelle dargestellt. Aus Beispiel 2 und 3 folgt, daß die Verringerung der Katalysatorkonzentration (Methylat des Alkalimetalls) bis zu 0,2 Ma.-% zu einer Verringerung der Produktivität des Methylformiates führt, bei Erhöhung bis zu 6 Ma.-% des Alkalimethylates beobachtet man eine Erhöhung des Absetzproduktes (Reaktionsprodukte des Methylates) und eine Erhöhung des Katalysatorverbrauches. Somit führt die Abweichung der Konzentration des Alkalimethylates von der genannten oberen und unteren Grenze zur Verschlechterung der grundlegenden Charakteristik des Prozesses.

Über die Effektivität der Anwendung der vorgeschlagenen Komponenten des katalytischen Systems (starke organische Basen und Natrium- oder Kalium-oxaperfluoralkansulfonate) wird in den Beispielen 4 bis 14 etwas ausgesagt. In allen diesen Beispielen erreicht man eine Erhöhung der Produktivität des Methylformiatprozesses (im Vergleich zum "Prototyp") bei gleichzeitiger Verringerung des spezifischen Verbrauches des Katalysators des Alkalimethylates.
In jedem der Beispiele 4 bis 14 erreicht man eine Verringerung des Anteils seiner aktiven Form CH₃OM im festen Niederschlag der Reaktionsprodukte des Katalysators, was aus der Erhöhung der Löslichkeit der Salze im Gemisch der Carbonylierung in Anwesenheit der vorgeschlagenen Zusätze resultiert.
Bei Anwendung einer optimalen Menge von Kalium-perfluor-5-methyl-3,6-dioxa-perfluoroctansulfonat (Beispiel 9) beträgt der spezifische Verbrauch des Katalysators 1,52 Kilogramm/Tonne Methylformiat.
Die Anwendung von N-Methyl-α-Pyrrolidon (5,0 Ma.-%, Beispiel 8) oder Guanidin (5,0 Ma.-% , Beispiel 11) als starke organische Basen gestattet es, die Produktivität für Methylformiat je Liter Reaktionsvolumen auf 296,7 Gramm/Stunde beziehungsweise 318,3 Gramm/Stunde zu erhöhen.

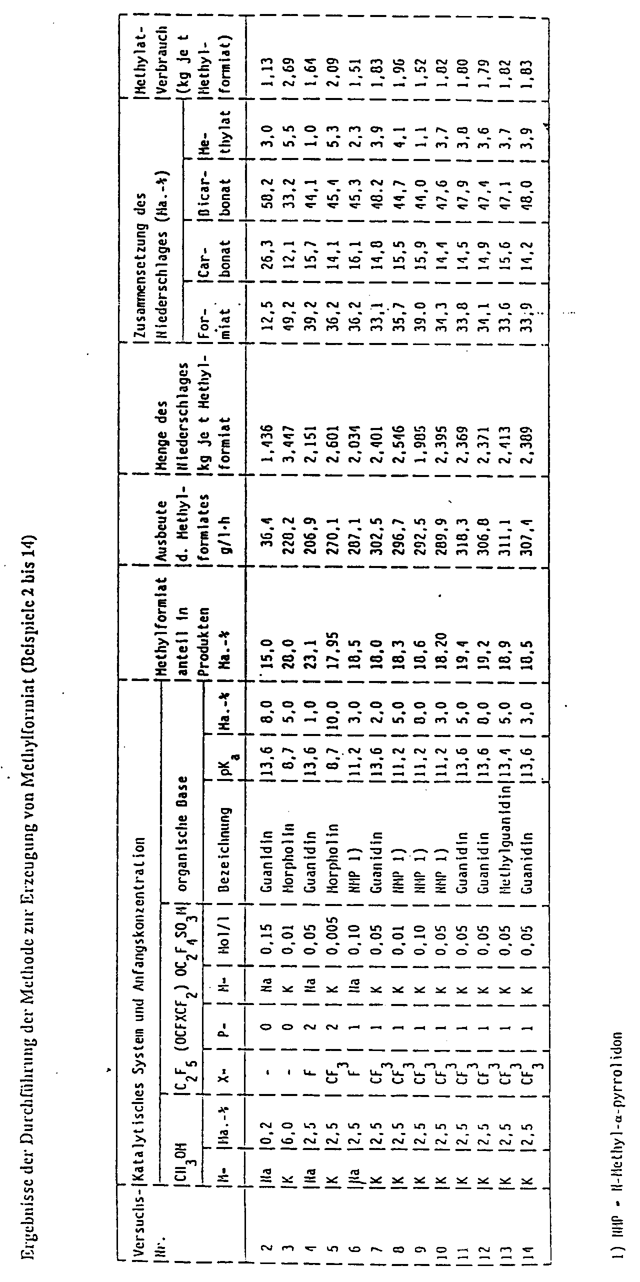

## Patentansprüche

1. Verfahren zur Erzeugung von Methylformiat durch Carbonylierung von Methanol im Beisein von 0,2 bis 6,0 Ma.-% von Natrium- oder Kaliummethylat als Katalysator bei erhöhter Temperatur und erhöhtem Druck, mit dem Ziel der Verringerung des Katalysatorverbrauches und der Intensivierung des Prozesses, dadurch gekennzeichnet, daß in das Reaktionsgemisch zur Carbonylierung 0,005 bis 0,15 Mol/Liter Natrium- oder Kalium-oxa-perfluoralkansulfonat der allgemeinen Formel
CF₃CF₂(OCFXCF₂)ₚOCF₂CF₂SO₃M
mit
p = 0 bis 2
X = F, CF3
M = Na, K
sowie 1 bis 10 Ma.-% starker organischer Basen mit pKa > 8,7 gegeben wird, wobei die Methylformiatkonzentration in den Produkten im Bereich von 15 bis 28 Ma.-% gehalten wird.

2. Verfahren zur Erzeugung von Methylformiat gemäß Anspruch 1, dadurch gekennzeichnet, daß man in das Reaktionsgemisch der Carbonylierung 0,01 bis 0,10 Mol/Liter des Kaliumsalzes der Perfluor-5-methyl-3,6-dioxa-perfluoroctansulfonsäure der Formel CF₃CF₂OCF(CF₃)CF₂OCF₂SO₃K gibt.

3. Verfahren zur Erzeugung von Methylformiat gemäß Ansprüchen 1 und 2, dadurch gekennzeichnet, daß dem Reaktionsgemisch der Carbonylierung 3 bis 8 Ma.-% N-Methyl-α-Pyrrolidon zugesetzt wird.

4. Verfahren gemäß Ansprüchen 1 und 2, dadurch gekennzeichnet, daß dem Reaktionsgemisch zur Carbonylierung 2 bis 8 Ma.-% Guanidin oder eines seiner Derivate zugesetzt wird.

## Claims

1. Method of producing methyl formate by carbonylising methanol in the presence of 0.2 to 6.0 % by wt. sodium or potassum methylate as the catalyst at a high temperature and at a high pressure, with the purpose of reducing the catalyst consumption and intensifying the process, characterised in that 0.005 to 0.15 mol/litre sodium- or potassium-oxa-perfluoroalkane sulphonate of the general formula
CF₃CF₂(OCFXF₂)ₚOCF₂CF₂SO₃M,
with
p = 0 to 2
X = F, CH₃
M = Na, K,
and 1 to 10 % by wt. strong organic bases with pKa > 8.7 are introduced into the reaction mixture for the carbonylisation, the methyl formate concentration in the products being kept within the range of between 15 and 28 % by wt.

2. Method of producing methyl formate according to claim 1, characterised in that 0.01 to 0.10 mol/litre of the sodium salt of perfluoro-5-methyl-3,6-dioxaperfluorooctane sulphonic acid of the formula CF₃CF₂OCF(CF₃)CF₂OCF₂SO₃K is introduced into the reaction mixture for the carbonylisation.

3. Method of producing methyl formate according to claims 1 and 2, characterised in that 3 to 8 % by wt. N-methyl-α-pyrrolidone is added to the reaction mixture for the carbonylisation.

4. Method according to claims 1 and 2, characterised in that 2 to 8 % by wt. guanidine or one of its derivatives is added to the reaction mixture for the carbonylisation.

## Revendications

1. Procédé de fabrication du formiate de méthyle par carbonylation du méthanol en présence de 0,2 à 6,0 % en masse d'éthylate de sodium ou de potassium utilisé comme catalyseur avec élévation de la température et de la pression, avec comme objectif une diminution de la consommation de catalyseur et l'augmentation du rendement du procédé, caractérisé en ce que dans le mélange de réaction de carbonylation sont ajoutés 0,005 à 0,15 mole/litre d'oxa-perfluoralcanesulfonate de sodium ou de potassium de la formule générale
CF₃CF₂(OCFXCF₂)ₚOCF₂CF₂SO₃M
où
p = 0 à 2
X = F, CF₃
M = Na, K
ainsi que 1 à 10 % en masse de bases organiques plus fortes avec pKa > 8,7, et où la concentration en formiate de méthyle dans les produits est maintenue dans la zone de 15 à 28 % en masse.

2. Procédé de fabrication du formiate de méthyle selon la revendication 1, caractérisé en ce que l'on ajoute au mélange de réaction de carbonylation contenant 0,01 à 0,10 mole/litre de sel de potassium, l'acide perfluor-5-méthyl - 3,6-dioxa-perfluorooctanesulfonique dont la formule est CF₃CF₂OCF(CF₃)CF₂OCF₂SO₃K.

3. Procédé de fabrication du formiate de méthyle selon les revendications 1 et 2, caractérisé en ce que 3 à 8 % en masse de N-méthylpyrrolidone-α sont ajoutés au mélange de réaction de carbonylation.

4. Procédé selon les revendications 1 et 2, caractérisé en ce que 2 à 8% en masse de guanidine ou de l'un de ses dérivés sont ajoutés au mélange de réaction de carbonylation.
